# EUROPEAN PATENT APPLICATION

(11) **EP 1 780 528 A1**
(43) Date of publication of application: **02.05.2007**
(21) Application number: 06255444.9
(22) Date of filing: 23.10.2006
(51) Int. Cl.: G01N 13/00, G01N 33/15

(54) **Dissolution test equipment and methods for testing having improved filtration system**

(30) Priority: 25.10.2005 US 729954 P
(71) Applicant: Rohm and Haas Company, Philadelphia, PA 19106-2399 (US)
(72) Inventor: Hughes, Lyn, Harleysville Pennsylvania 19438 (US)
(74) Representative: Kent, Venetia Katherine

(57) **Abstract**

The present invention provides a dissolution test apparatus comprising, a first chamber 2 connected in series to at least a second chamber 10, wherein the first chamber 2 is capable of transferring solid test samples to the second chamber 10 and the second chamber 10 is capable of retaining the solid test samples. Further, the apparatus includes a first reservoir 21 and at least a second reservoir 22 for continuously supplying one or more media into the first chamber 2 and the second chamber 10, respectively, the chambers having stirrers 4, 11 for mixing the solid test samples and the media together. In addition, the second chamber 10 has a filter membrane 45 overlying a base 44 of the second chamber 10, the filter membrane 45 being supported by a filter support 46, the filter support 46 being provided between the filter membrane 45 and the base 44 of the second chamber 10 to prevent distortion of the filter membrane 45. Processors 5, 16, 20 for analyzing an effluent from the chambers are also provided.

## Description

### BACKGROUND OF THE INVENTION

The rate at which pharmaceutically active compounds dissolve in gastrointestinal fluids is crucial in the design and use of orally administered medications. The active compound must be dissolved before it can be absorbed by the body. The rate at which the active substance enters into solution is know in the art as the dissolution rate, and the determination of the dissolution rate in vitro is known as dissolution testing.

Dissolution testing provides a better understanding of the amount of a pharmaceutically active compound available at a particular absorption site at various times. In addition, establishing a relationship between dosage form and availability of a pharmaceutically active compounds at certain absorption sites and systemic blood levels of such active compound aids in the development of specialized delivery techniques.

The concept of using in vitro data to predict or model in vivo behavior, referred to as in vitro-in vivo correlation, or IVIVC, is of great interest in the pharmaceutical arts. Test methods with good IVIVC are better for detecting problems with existing formulations and in the development of new formulations. Systems that correlate closely with the dissolution and absorption data obtained in vivo can be used in developing dosage forms as well as in the production, scale-up, determination of lot-to-lot variability, testing of new dosage strengths, testing of minor formulation changes, testing after changes in the site of manufacture and for determining bio-equi valence.

In U.S. Pat. No. 4,335,438 to Smolen, it describes a dissolution test method utilizing a combination of a flow-through cell (with recycle) and a mathematical model. The mathematical model is used in conjunction with varying test parameters in order to provide an optimized in vivo plasma curve from the in vitro data. In Smolen, there are four basic variables, flow rate, amount of recycle, pH, and stirring rate that are necessarily changed with time so that the number of possible combinations of variables is increased. Unfortunately, the results obtained using the Smolen cell, based on USFDA guidelines, are not an acceptable for establishing IVIVC because the number of independent variables are too great. In other words, the dissolution test method of Smolen may not be physiologically relevant. In addition, the flow-through system of Smolen utilizes only one cell per test, further compromising the IVIVC.

Thus, there is a need for an integrated assessment of in vitro dissolution of a pharmaceutical formulation and absorption of an active compound from such formulation, especially with the development of more advanced dosage forms, for example, formulations that provide a delayed release of a compound. Also, there is need for an in vitro dissolution test that takes into account the absorption of the active substance by the body and the presence of dissolved active substance during the dissolution. Further, there is also a need for an in vitro dissolution test that is able to demonstrate Level A IVIVC without the need for mathematical models to transform the in vitro data.

### STATEMENT OF THE INVENTION

In a first aspect of the present invention, there is provided a dissolution test apparatus comprising: a first chamber connected in series to at least a second chamber, wherein the first chamber is capable of transferring solid test samples to the second chamber and the second chamber is capable of retaining the solid test samples; a first reservoir and at least a second reservoir for continuously supplying one or more media into the first chamber and the second chamber, respectively, the chambers having a stirrer for mixing the solid test samples and the media together; wherein the second chamber has a filter membrane overlying a base of the second chamber, the filter membrane being supported by a filter support, the filter support being provided between the filter membrane and the base of the second chamber to prevent distortion of the filter membrane; and a processor for analyzing an effluent from the chambers.

In another aspect of the present invention, there is provided a dissolution test apparatus comprising: a first chamber connected in series to at least a second chamber, wherein the first chamber is capable of transferring solid test samples to the second chamber and the second chamber is capable of retaining the solid test samples; a first reservoir and at least a second reservoir for continuously supplying one or more media into the first chamber and the second chamber, respectively; a filter membrane provided in a base of the first and second chambers, the base having ridges formed thereon; a filter support provided over the ridges to prevent distortion of the filter membrane; and a processor for analyzing an effluent from the chambers.

In another aspect of the present invention, there is provided a method for dissolution testing comprising: transferring solid test samples from a first chamber connected in series to at least a second chamber, wherein the second chamber is capable of retaining the solid test samples; continuously supplying one or more media into the first chamber and the second chamber from a first reservoir and at least a second reservoir, respectively; mixing the solid test samples and the media together; filtering the solid test samples through a filter membrane supported by a filter support, the filter support overlying a base of the first and second chambers to prevent distortion of the filter membrane; and analyzing an effluent from the chambers.

### BRIEF DESCRIPTION OF THE DRAWINGS

Figure 1 illustrates a dissolution test apparatus of the present invention;

Figure 2 illustrates a dip-tube and Tee assembly utilized in the apparatus of the present invention;

Figure 3 illustrates a top-plan view of the base of the cell utilized in the apparatus of the present invention; and

Figure 4 illustrates a side-sectional view of the base of the cell utilizing the filter support of the present invention.

### DETAILED DESCRIPTION OF THE INVENTION

The present invention provides a new dissolution testing apparatus and methods for testing that incorporate disintegration, solids transfer, dissolution, changing pH/composition of fluids, absorption, and clearance. The present invention provides excellent Level A IVIVC and is predictive across different dosage forms of the same drug. In addition, no mathematical model is required. Also, the present invention provides an improved filtration system including a filter support that prevents the filter membrane from deforming or distorting (from the increased pressure) into the molded shape of the ridges on the base of the cell. According to the invention, the addition of the filter support prevents the distortion of the filter membrane and allows for the continuous, unrestricted flow of the media through the cells and throughout the apparatus.

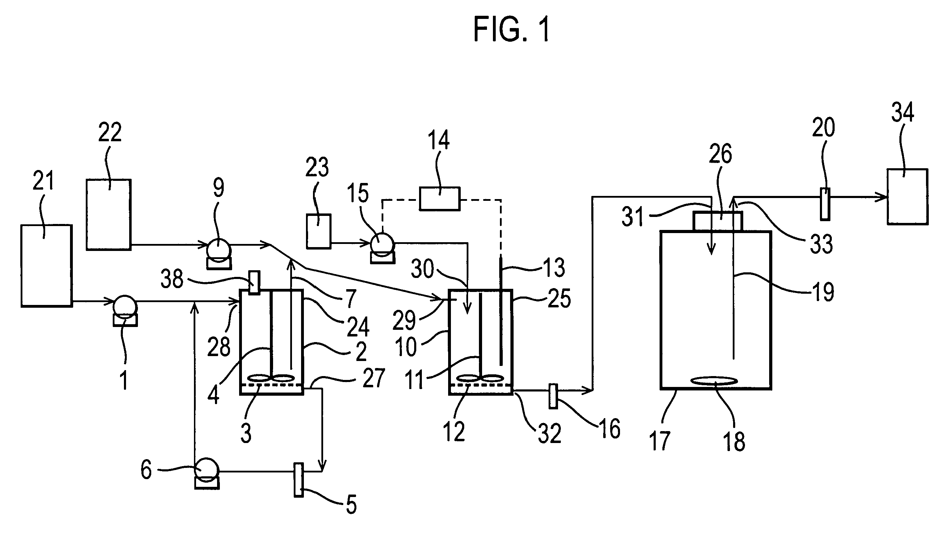

Referring now to the drawings, Figure 1 illustrates one embodiment of the dissolution apparatus of the present invention. A reservoir 21, a pump 1, and a filtration cell 2 are connected such that the liquid contents (media) of the reservoir 21 is transferred into the filtration cell 2 via the pump 1. The filtration cell 2 is equipped with a tight fitting lid 24, a filtration membrane 3, a stirrer 4, an inlet 28, an outlet 47 positioned to allow removal of filtered liquid, a sample holder 38, and a dip-tube and Tee assembly 7. The outlet 47 is connected to a flow-thru uv cell 5 and pump 6, such that the filtrate is pumped through the uv cell 5 and returned to the inlet 28 of the filtration cell 2. One branch of the dip-tube and Tee assembly 7 comprises a dip-tube to allow removal of liquid and small particle sized solids from the filtration cell 2. The second branch of the dip tube and Tee assembly is connected to the outlet of a pump 9. The third branch of the dip-tube and Tee assembly 7 is connected to the inlet 29 of a second filtration cell 10.

A reservoir 22 is connected to the pump 9 such that the liquid media from the reservoir 22 is fed into the second branch of the dip-tube and Tee assembly 7. The filtration cell 10 is equipped with a tight fitting lid 25, a pH sensor 13, a stirrer 11, a filtration membrane 12, two inlets 29 and 30 , and an outlet positioned to allow removal of filtered liquid. A reservoir 23 is connected to a pump 15 and to one of the inlets 30 of the filtration cell 10, such that liquid from the reservoir 23 is transferred into the filtration cell 10. The outlet 47 is connected to a flow-thru uv cell 16. The outlet of the uv cell 16 is connected to the inlet 31 of the cell 17. The pH sensor 13 is electrically connected to a pH controller 14. The power supply to pump 15 is connected to the output relay of the pH controller 14 such that the pump 15 is turned on when the pH, as measured by the pH sensor 13, is below a target value, and is turned off when the pH is above a target value.

The cell 17 is equipped with a tight fitting lid 26, a stirrer 18, a dip-tube 19, and an outlet 33. The outlet is connected to the inlet of a flow-thru uv cell 20. The outlet from the uv cell 20 is directed to waste or any suitable reservoir 34. In this embodiment, the filtration cell 2 and immediately associated equipment represents the gastric chamber; the filtration cell 10 and immediately associated equipment represents the intestinal chamber; the cell 17 and immediately associated equipment represents the circulatory chamber. Each of the flow-thru uv cells 5, 16, and 20 is placed in a suitable uv spectrophotometer capable of measuring the absorbance of the cell contents at the desired wavelength.

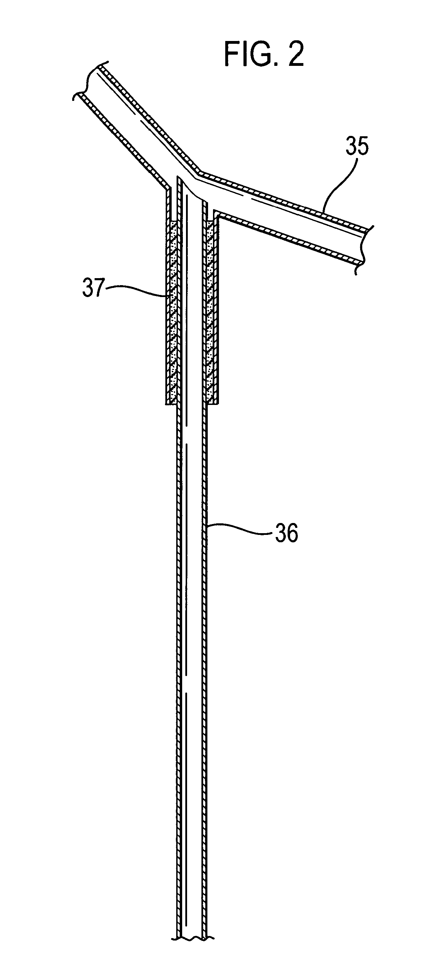

Figure 2 schematically illustrates one embodiment of the mixing device herein called a dip-tube and Tee assembly. The dip-tube and Tee assembly 7 comprises a Y-shaped connector 35, a length of tubing 36, and a sealant 37. The tubing 36 is inserted into one of the branches of the connector 35 together with a sealant to prevent leakage of liquid media and to hold the tubing in place during operation. The length of the tubing is adjusted so that the lower end is below the surface of the liquid when the equipment is operating. This arrangement allows the transfer of small particles between the gastric (first chamber) and intestinal chambers (second chamber) without clogging the tubing. Large particles are not transferred by this arrangement because they are too large to enter the dip tube or because the flow velocity of the medium in the dip tube is insufficient to carry the large particles up the dip tube.

When control of the temperature is required any or all of the three cells can be immersed in a suitable heating bath or a recirculating hot air oven. In one embodiment, reservoir 21 is filled with simulated gastric fluid, reservoir 22 is filled with simulated intestinal fluid, and reservoir 23 is filled with 0.8M aqueous sodium hydroxide solution. To start a test, the pumps are operated to fill each of the chambers to the desired volumes, and then run for sufficient time to establish that the flow rate from each pump is as desired, the temperature is as desired and the pH of cell 10 is maintained within the target range. The uv cells are checked to make sure that they contain no air bubbles. In one embodiment, the sample in the sample holder 38 is lowered into the filtration cell 2, down to a fixed distance within the cell 2. Note, the sample is introduced into the cell 2 without stopping/starting the pumps. Exposure to the fluid in the gastric chamber causes the sample to be partially or completely disintegrated, or dispersed or dissolved. The dissolved portion exits the gastric chamber via the tube 36 together with small particles of undissolved drug and/or excipient. Dissolved drug and/or dissolved excipient also exits the gastric chamber through the outlet 47. The filter membrane 45 prevents undissolved particles from exiting through the outlet 47. The liquid that exits though outlet 47 passes through the uv cell 5, where it's uv absorbance at any desired wavelength is continuously monitored. The liquid is continuously returned to the gastric chamber via the inlet 28. The material exiting via the tube 36 enters the Tee 35 where it mixes with simulated intestinal fluid from the pump 9. This mixture then enters the simulated intestinal chamber via the inlet 29.

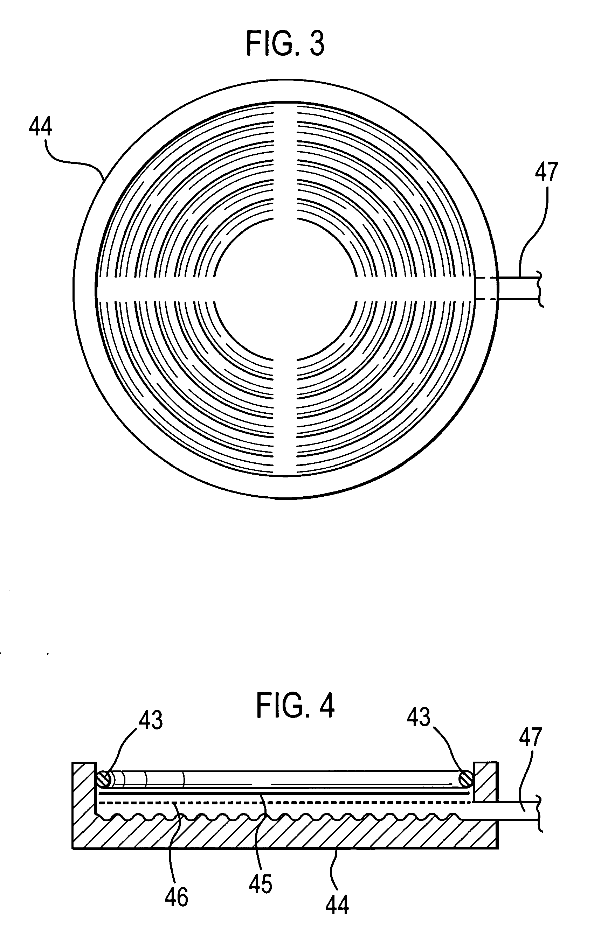

Referring now to Figure 3, there is shown a top-plan view of the base 44 of the cell 2 of the present invention. As shown, the base 44 has ridges 48 to promote the free flow of the media that is received from the reservoir 21, and pumped out through the outlet 47. In other words, the ridges 48 provide voids or spaces, in which the media can gather or collect and then exit through the outlet 47. Restricting or reducing the voids or spaces will diminish the media flow, and perhaps cause complete blockage. Note, although the ridges 48 are shown in a circular pattern, any desired pattern that promotes media/solvent flow is acceptable.

Figure 4 illustrates a side-sectional view of the base 44 of the cell 2 of the present invention. As illustrated, the filter membrane 45 is provided over the base of the cell 44, in particular, over ridges 48. The filter membrane 45 is firmly held in place by a retainer ring, preferably, an O-ring. The media flows through the filter membrane 45, into the ridges 48 and then exits toward the outlet 47. Advantageously, the filter support 46 is provided between the ridges 48 and the filter membrane 45, preventing the filter 45 from deforming or distorting (from the increased pressure) into the molded shape of the ridges 48. In other words, as the pressure increases within the system, there is a tendency for the filter 45 to be pulled into the base of the cell 44. In particular, there is a tendency for the filter 45 to "fill-in" the ridges 48 of the base of the cell 44, impeding or restricting the flow of the media within the ridges 48. However, according to the invention, the addition of the rigid filter support 46 prevents the distortion of the filter membrane 45 and allows for the continuous, unrestricted flow of the media through the cell 2 and throughout the apparatus. The filter support 46 may be any material that is at least less flexible than the filter membrane 45. Preferably, the support 46 is a metal mesh screen.

In the intestinal chamber, the incoming mixture is mixed with the contents of the chamber together with sodium hydroxide solution entering from pump 15. Because the sodium hydroxide flow is controlled by the pH of the contents of the cell 10 the result is that the acid present in the gastric fluid portion of the incoming mixture is neutralized. In the intestinal chamber the undissolved portion of the incoming mixture has further opportunity to dissolve. Dissolved drug and/or dissolved excipient exits the intestinal chamber through the outlet 47. The filter membrane 45 prevents any undissolved drug and/or undissolved excipient from exiting the chamber. The liquid that exits though outlet 47 passes through the uv cell 16, where it's uv absorbance at any desired wavelength is continuously monitored. The liquid exiting the uv cell 16 then enters the circulatory chamber via the inlet 31.

Note, the intestinal chamber (cell 10) is also provided with the filter support 46 of the present invention. Referring back to Figure 3, there is also shown a top-plan view of the base 44 of the cell 10 of the present invention. As shown, the base 44 has ridges 48 to promote the free flow of the media that is received from the reservoir 22, and pumped out through the outlet 47. In other words, the ridges 48 provide voids or spaces, in which the media can gather or collect and then exit through the outlet 47. Restricting or reducing the voids or spaces will diminish the media flow, and perhaps cause complete blockage. Note, although the ridges 48 are shown in a circular pattern, any desired pattern that promotes media/solvent flow is acceptable.

Also, referring back to Figure 4, there is shown a side-sectional view of the base 44 of the cell 10 of the present invention. As illustrated, the filter membrane 45 is provided over the base of the cell 44, in particular, over ridges 48. The filter membrane 45 is firmly held in place by a retainer ring, preferably, an O-ring. The media flows through the filter membrane 45, into the ridges 48 and then exits toward the outlet 47. Advantageously, the filter support 46 is provided between the ridges 48 and the filter membrane 45, preventing the filter 45 from deforming or distorting (from the increased pressure) into the molded shape of the ridges 48. In other words, as the pressure increases within the system, there is a tendency for the filter 45 to be pulled into the base of the cell 44. In particular, there is a tendency for the filter 45 to "fill-in" the ridges 48 of the base of the cell 44, impeding or restricting the flow of the media within the ridges 48. However, according to the invention, the addition of the rigid filter support 46 prevents the distortion of the filter membrane 45 and allows for the continuous, unrestricted flow of the media through the cell 10 and throughout the apparatus. The filter support 46 may be any material that is at least less flexible than the filter membrane 45. Preferably, the support 46 is a metal mesh screen. Note, although invention has been described with a filtration system (including a filter support 46) in both the gastric chamber (cell 2) and the intestinal chamber (cell 10), typically, a filtration system is solely provided in the intestinal chamber to prevent any solids from entering the circulatory system (cell 17).

In the circulatory chamber, the incoming medium is mixed with the medium already present in the chamber. The resulting mixture continuously exits the chamber via the dip-tube 19 and outlet 33. The liquid that exits though outlet 33 passes through the uv cell 20, where the uv absorbance at any desired wavelength is continuously monitored. The data collected from the spectrophotometer can be used to calculate the instantaneous concentration of the active substance. The data can be used to characterize the release rate and the total amount of active substance released. Measuring the concentration of active substance in the effluent collected in the collection reservoir 34 permits the calculation of the total amount of active substance released.

While the embodiment of the invention described above uses constant composition of release fluids, the compositions can be changed with time to simulate changing conditions within the body. Test method variables are, for example, composition of release media, residence time in each of the three chambers, amount of the sample being tested, and temperature. By adjusting these variables it is possible to obtain a release rate profile that matches the plasma concentration profile observed in vivo. When practiced in the pharmaceutical industry, the preferred temperature is 37° C., and the preferred composition of the release media are simulated gastric and simulated intestinal fluids.

Also, other additives, such as enzymes, bile acids, and surfactants, can be included, as desired. In addition, although the USFDA recommends that dissolution conditions be physiologically relevant, the present invention can be adapted for conditions that are not physiologically relevant. Such conditions may be desirable when considerations such as speed of operation, unusual solubility, or non conventional dosage forms are taken into account. For example, applicant has determined in some cases that by proportionally reducing residence times, the time scale of the test can be considerably shortened without loss of useful information. In addition, the invention can be used to test many different types of formulations. These can include, but are not restricted to, tablets, powders, pills, syrups; fast-melt tablets, hard capsules and soft capsules.

The medium analysis device includes, but is not limited to, any detector known in the art that generates physical and/or chemical data of a pharmaceutical or active test agent, e.g., the use of a UV spectrophotometer as the method of analysis. In a preferred embodiment, the detector is capable of acquiring data characteristic of a particular agent by any method, including, ultraviolet radiation, infrared radiation, nuclear magnetic resonance, Ramen spectroscopy, electrochemical, biosensors, refractometry, optical activity, and combinations thereof. Also, any in-line detector known in the art that is applicable to the active substance and release medium can be used. Preferably, the medium dissolution analysis device is a detector that has a sensor communicatively attached thereto. In the preferred embodiment, there is at least one medium dissolution analysis device per dissolution chamber. For example, for each sample to be analyzed there is a corresponding medium dissolution analysis device capable of continuously generating physical and/or chemical data characteristic of the agent to be analyzed.

The medium analysis device preferably includes a detector operatively associated with the dissolution medium for at least the time period required for the dosage form to release the maximum releasable quantity of therapeutically active agent and a data processor for continually processing the generated data for at least the time period required for the dosage form to release the maximum releasable quantity of therapeutically active agent to obtain a dissolution profile of the dosage form. The data processor may be any device capable of continuously processing the data generated by the detector. In a preferred embodiment, the data processor is a computer. The data generated by the detector is preferably stored and/or analyzed by the computer. In a particularly preferred embodiment, the data collector is a computer that has data processing software. The data is preferably continuously processed by the software as it is received from the detector.

In the preferred embodiment of the present invention, the detector measures the concentration of the therapeutically active agent in the media surrounding the dosage form such as in simulated gastric or intestinal fluid. By measuring the concentration of the agent in the surrounding media, the amount of agent released from the dosage form can be calculated. The invention can also be used by removing samples from the chambers directly or from the effluent discharge of the chambers instead of, or in addition to in-line analysis. In such an embodiment the analytical methods can be any method known in the art, including but not limited to, Gas chromatography, liquid chromatography, high performance liquid chromatography (HPLC), colorimetry, uv spectroscopy, IR spectroscopy, Raman spectroscopy, near IR spectroscopy, bio-sensors, electrochemical methods, mass spectroscopy, and nuclear magnetic spectroscopy. In the most preferred embodiment the medium analysis is performed in- line using uv spectroscopy.

In the present invention, any combination of the medium analysis devices can be used as appropriate for the data required. In another embodiment the absorption of active substance in the stomach can be simulated by not returning to the gastric chamber, all or part of the medium exiting the gastric chamber via the second outlet. The flow rate of the medium can be adjusted so that the removal rate corresponds to the in vivo gastric absorption.

The filtration cells 2 and 10 can be of any design that provides the requirements of agitation, desired volume, filtration speed, filtration efficiency, and compatibility with the active substance and the release media. The preferred filtration cells are continuous and stirred, filtration cells (e.g., Amicon stirred ultrafiltration cell models 8003, 8010, 8050, 8200, and 8400 available from Millipore Corporation).

The third cell 17 can be of any design that provides the requirements of agitation, desired volume, and compatibility with the active substance and the release media. The pumps useful in the practice of the present invention can be any pump capable of attaining the desired flow rate and maintaining the flow rate constant throughout the test. These include but are not limited to, general purpose positive displacement pumps, peristaltic pumps, diaphragm pumps, HPLC quality positive displacement pumps, syringe pumps and centrifugal pumps. Preferred pumps useful in the invention are peristaltic pumps, diaphragm pumps, and HPLC quality positive displacement pumps. Most preferred are peristaltic pumps and HPLC quality positive displacement pumps.

Heating devices useful in the practice of the present invention can be any of those known in the art that give sufficiently uniform and accurate temperature control. The preferred heating device will be able to control the temperature to within +/-2° C. of the desired temperature. The more preferred heating device will be able to control the temperature to within +/-1° C. of the desired temperature. The most preferred heating device will be able to control the temperature in conformity with the most current recommendations in the US Pharmacopeia and like sources.

The tubing used in the dip-tube and Tee assembly can be any tubing compatible with the release medium and the test sample. The length of the tubing is adjusted such that the lower end is below the surface of the liquid in the filtration cell 2. The cross-sectional diameter of the tubing is selected so that small particles are carried up the tubing by the flow of the release medium and so that particles do not clog the tubing. In practice, the inventors have determined that tubing with an internal diameter of 0.5 to 3.0 mm fulfills these requirements for flow rates to the cell 2 in the range 0.5 to 2.5 m/min. For other flow rates other internal diameters may be needed.

The medium analysis sensor and controller used with the intestinal chamber can be any combination of sensor and controller that measures and permits control of physical characteristics such as, but not limited to, pH, osmolarity, conductivity, and concentration of specific ions. The preferred medium analysis sensor and controller are any pH sensor and pH controller available in the art that permit the control of the pH in the intestinal chamber to within the target range. The most preferred medium analysis sensor and controller are any pH sensors and pH controllers available in the art that has an accuracy of +/-0.02 pH units.

In the preferred embodiment the pH in the second cell 10 is controlled to the same value as that of the simulated intestinal fluid. Also, the pH in the the cell can be any value achievable by addition of either an acid or a base through the delivery system defined by the reservoir 23, the pump 15, and the inlet 30, and is not limited to the pH of the fluid in the reservoir 22. The solution used to adjust the pH of the second cell 10 can be acidic or basic. The preferred concentration of acid or base in the solution is one that requires a flow rate of the solution to be not more than 10% of the total flow of the other release media. The most preferred concentration of acid or base in the solution is one that requires a flow rate of the solution to be not more than 2% of the total flow of the other release media.

The number of cells used in the equipment can be varied depending on the information required. Three cells, as described in one embodiment above, is the preferred number when correlation with blood plasma concentration data is required. When drug absorption rate data is required it is only necessary to operate the combination of gastric and intestinal chambers. A further possibility is to add a buccal dissolution cell before the gastric chamber such that the effluent from the buccal dissolution chamber enters an inlet in the gastric chamber. Note, the sample holder would be present on the buccal dissolution chamber, not the gastric chamber. The addition can be used for either drug absorption or blood plasma concentration data.

In addition, filter membranes useful in the practice of this invention can be any of the commercially available filter membranes that are compatible with the release media. Preferred filter membranes have a nominal particle size cut-off of not more than 10 microns. The more preferred filters have a nominal particle size cut-off of 0.25-5 microns. The most preferred filter membranes have a nominal particle size cut-off of 1-3 microns.

Residence times in each of the chambers useful in the practice of this invention can be any value required to give a Level A IVIVC. The preferred residence times are those that have physiological relevance. The Applicants have determined by experimentation that the following ranges of residence times are useful: gastric chamber, 5-60 minutes; intestinal chamber, 1-90 minutes; circulatory chamber, greater than 30 minutes. In addition, various other mechanical, electrical and electronic equipment may be incorporated into the present invention. For example, the equipment includes, but is not limited to, pressure relief valves, check valves, pressure relief piping, pressure control systems, surge suppressors, surge tanks, de-aerators, electronic flow control systems, proportional control systems, pressure gauges, heat exchanges (to preheat media) and flow gauges.

Accordingly, the present invention provides a new dissolution testing apparatus and methods for testing that incorporate disintegration, solids transfer, dissolution, changing pH/composition of fluids, absorption, and clearance. The present invention provides excellent Level A IVIVC and appears to be predictive across different dosage forms of the same drug. In addition, no mathematical model is required. Also, the present invention provides a filter support that prevents the distortion of the filter membrane and allows for the continuous, unrestricted flow of the media through the cells and throughout the apparatus, resulting in a more reliable and accurate IVIVC.

## Claims

1. A dissolution test apparatus comprising:
a first chamber connected in series to at least a second chamber, wherein the first chamber is capable of transferring solid test samples to the second chamber and the second chamber is capable of retaining the solid test samples;
a first reservoir and at least a second reservoir for continuously supplying one or more media into the first chamber and the second chamber, respectively, the chambers having a stirrer for mixing the solid test samples and the media together;
wherein the second chamber has a filter membrane overlying a base of the second chamber, the filter membrane being supported by a filter support, the filter support being provided between the filter membrane and the base of the second chamber to prevent distortion of the filter membrane; and
a processor for analyzing an effluent from the chambers.

2. The apparatus of claim 1 wherein the first chamber has another filter membrane overlying a base of the first chamber, the another filter membrane being supported by another filter support, the another filter support being provided between the another filter membrane and the base of the first chamber to prevent distortion of the another filter support.

3. The apparatus of claim 1 further comprising an O-ring to secure the filter membrane to the base of the second chamber.

4. The apparatus of claim 1 wherein the filter support is a metal mesh screen.

5. The apparatus of claim 1 wherein the first chamber comprises simulated gastric fluid.

6. The apparatus of claim 1 wherein the second chamber comprises simulated intestinal fluid.

7. The apparatus of claim 1 further comprising a third chamber connected in series to the second chamber for receiving the effluent from the second chamber.

8. The apparatus of claim 1 further comprising a third reservoir connected to the second chamber for supplying a solution for adjusting a pH of the media in the second chamber.

9. A dissolution test apparatus comprising:
a first chamber connected in series to at least a second chamber, wherein the first chamber is capable of transferring solid test samples to the second chamber and the second chamber is capable of retaining the solid test samples;
a first reservoir and at least a second reservoir for continuously supplying one or more media into the first chamber and the second chamber, respectively;
a filter membrane provided in a base of the first and second chambers, the base having ridges formed thereon;
a filter support provided over the ridges to prevent distortion of the filter membrane; and
a processor for analyzing an effluent from the chambers.

10. A method for dissolution testing comprising:
transferring solid test samples from a first chamber connected in series to at least a second chamber, wherein the second chamber is capable of retaining the solid test samples;
continuously supplying one or more media into the first chamber and the second chamber from a first reservoir and at least a second reservoir, respectively;
mixing the solid test samples and the media together;
filtering the solid test samples through a filter membrane supported by a filter support, the filter support overlying a base of the first and second chambers to prevent distortion of the filter membrane; and
analyzing an effluent from the chambers.
